# EUROPÄISCHE PATENTSCHRIFT

(11) **EP 1 698 307 B1**
(45) Veröffentlichungstag und Bekanntmachung des Hinweises auf die Patenterteilung: **15.09.2010**
(21) Anmeldenummer: 06003788.4
(22) Anmeldetag: 24.02.2006
(51) Int. Cl.: A61F 2/46, A61B 17/16

(54) **Medizinisches Instrument zur autologen Chondrozytentransplantation**
Medical instrument for autologous chondrocyte transplantation
Instrument médical pour la transplantation de chondrocytes autologues

(30) Priorität: 03.03.2005 DE 102005010988
(43) Veröffentlichungstag der Anmeldung: 06.09.2006
(73) Patentinhaber: Karl Storz GmbH & Co. KG, 78532 Tuttlingen (DE)
(72) Erfinder: Steinwachs, Matthias, Dr., 79112 Freiburg (DE); Berberich, Sascha, 78532 Tuttlingen (DE)
(74) Vertreter: Weller, Wolfgang

(56) Entgegenhaltungen:
- EP-A- 0 824 893
- WO-A-00/09179
- US-A1- 2004 015 170

## Beschreibung

Die Erfindung betrifft ein Instrument zur autologen Chondrozytentransplantation, mit einem Stempel zum Einbringen eines Implantatvlieses in einen Knorpel, der an seiner distalen Endfläche zumindest eine Öffnung aufweist, die mit einem Unterdruck beaufschlagbar ist, so dass ein Implantatvlies an der Endfläche haltbar ist.

Aus der DE 92 16 033 U ist ein Speichelzieher für den zahnärztlichen Gebrauch bekannt. Der Speichelzieher besteht aus einem Rohr, auf dessen distales Ende eine mit Durchbrechungen versehene Kappe aufsteckbar ist. An das proximale Ende ist eine Vorrichtung zur Erzeugung von Unterdruck anschließbar, so dass, wenn der Speichelzieher in die Mundhöhle eines Menschen eingelegt ist, aus dieser überflüssiger Speichel abgezogen wird.

Die autologe Chondrozytentransplantation (ACT) ist ein Verfahren zur Reparatur von Knorpelschäden im menschlichen Körper.

Der Gelenkknorpel im Kniebereich des Menschen ist je nach Topographie unterschiedlich dick. Im Bereich der Patella kann er eine Schichtdicke von 7 bis 8 mm erreichen. Da der Gelenkknorpel keinen unmittelbaren Gefäß- oder Nervenanschluss besitzt, erfolgt seine Ernährung überwiegend durch Diffusion aus der Synovialflüssigkeit des Gelenkbinnenraums. Die Vernetzung unterschiedlicher Matrixkomponenten zur Knorpelgrundsubstanz ermöglicht eine mechanische Dämpfung und das fast reibungslose Gleiten der Gelenkflächen. Feingeweblich findet sich ein komplexer Aufbau aus Knorpelzellen (Chondrozyten), Collagenfasern und Proteoglykanen. Der gesunde Knorpel im Kniebereich des erwachsenen Menschen ist in der Lage Belastungskräfte zu tolerieren, die ein Vielfaches des Körpergewichts betragen können.

Verletzungen des Gelenkknorpels stellen ein großes Problem im traumatologisch-orthopädischen Alltag dar. Das eingeschränkte Heilungsvermögen von Knorpel ist seit langem bekannt und ist im Wesentlichen in seiner besonderen Struktur und Anatomie begründet.

Ein Schaden an der Gelenkoberfläche, vor allem im Bereich der Belastungszonen der Gelenklauffläche, birgt daher das erhöhte Risiko einer weitergehenden Gelenkschädigung im Sinne einer frühzeitigen Arthrose. Bekannte Verfahren zur biologischen Rekonstruktion eines vollschichtigen Knorpelschadens sind meist nur für kleine bis mittlere Defektgrößen geeignet. Bei vollschichtigen Knorpelschäden, vor allem im Bereich des Kniegelenkes, mit mehr als ca. 4 cm² Defektausbreitung findet daher zunehmend die autologe Chondrozytentransplantation klinische Anwendung.

Bei diesem Verfahren wird auf arthroskopischem Wege Knorpelgewebe aus nicht tragenden Gelenkanteilen entnommen. Daraus werden Knorpelzellen isoliert und in einer Zellkultur angezüchtet.

Diese Anzüchtung der Knorpelzellen erfolgt immer häufiger innerhalb eines vliesartigen Implantats, z.B. einer Collagenmatrix. Ein solches Vlies stellt den Knorpelzellen eine dreidimensionale Struktur zur Verfügung, auf der sie wachsen und sich ausbreiten können.

Ein so erhaltenes mit Chondrozyten besetztes Vlies wird dann während eines zweiten Eingriffs in die Knorpeldefektzone transplantiert.

Während dieses zweiten Eingriffs werden die Ränder des Knorpeldefekts präpariert, d.h. es werden die Ränder des defekten Bereichs herausgeschnitten und der Knochen im Bereich des Defekts freigelegt. Hierbei ist besonders darauf zu achten, dass die Ränder des Defektbereichs sauber präpariert sind, um ein reibungsloses Anwachsen des Implantats zu ermöglichen.

Üblicherweise wird nun aus einem sterilen Material eine Schablone des Defekts angefertigt und mit Hilfe dieser Schablone das Implantatvlies zugeschnitten. Dieses so zugeschnittene Implantatvlies wird dann in den Knorpeldefekt eingesetzt.

Diese Prozedur erfolgt im Allgemeinen arthrotomisch, also mittels einer halboffenen Prozedur. Eine solche halboffene Prozedur birgt stets die Möglichkeit einer Infektion an der freiliegenden Operationsstelle. Außerdem wäre es weniger belastend für den Patienten, wenn eine solche Operation mittels minimalinvasiver Techniken, also auf arthroskopischem Wege durchgeführt wird.

Die US 2004/0015170 A1 betrifft ein System und ein Verfahren zur Reparatur von Gelenkoberflächen. In diesem Dokument ist eine Saugvorrichtung offenbart, die zum Halten eines Implantats mittels Saugkraft bei dessen Einbringen an einer Operationsstelle dient. Die Saugvorrichtung weist an ihrem distalen Abschnitt einen aus einem Elastomer ausgebildeten Saugkopf auf, der mit einer einzigen Öffnung versehen ist. Die Saugvorrichtung weist einen Anschluss auf, der mit einer Quelle für einen Unterdruck verbindbar ist. Mittels dieses Anschlusses wird die Öffnung der Saugvorrichtung mit einem Unterdruck beaufschlagt. Dadurch wird ein Implantat an den Saugkopf angesaugt, und somit wird er bei dessen Einbringen an die Operationsstelle an dem Saugkopf fixiert.

Die WO 00/09179 betrifft Verfahren und Vorrichtungen zur Chondrozytentransplantation. Ein in diesem Dokument offenbarter arthroskopischer Einführer weist eine Hülse mit einem Arbeitskanal auf, in dem eine zweite Hülse mit einem Injektionskanal angeordnet ist. Die zweite Hülse ist mit einem Griff verbunden, dessen teleskopische Bewegung eine Bewegung der zweiten Hülse in dem Arbeitskanal der ersten Hülse bewirkt. Ferner weist der Einführer zwei Elemente auf, die zum Halten eines Implantats bei dessen Einführung und Platzierung dienen. Die Elemente sind mit dem Griff verbunden und durch dessen Bewegung betätigbar. Bei einer Transplantation wird ein gerolltes Implantat durch die Elemente im Inneren des Einführers gehalten. Dann wird der Einführer mit dem Implantat an gewünschte Operationsstelle gebracht und dort wird das Implantat in den Knorpeldefekt eingesetzt.

Die EP 0 824 893 A2 betrifft eine Vorrichtung zur Transplantation von Knochen-Knorpelautotransplantaten. In diesem Dokument ist ein Transplantat-Gewinnungsgerät offenbart, das ein Rohr aufweist, das an einem kanulierten Handgriff angebracht ist. Das Rohr ist an seinem distalen Ende mit einer scharfen Schneidkante versehen, die zum Stanzen von Implantaten, beispielsweise Knorpelimplantaten oder Implantatvliesen, dient. Bei der Verwendung des Transplantat-Gewinnungsgeräts wird das Rohr mit einem angepassten Durchmesser vollständig in einen Treiber für das rohrförmige Gewinnungsgerät eingeführt und an eine Operationsstelle gebracht. Dann wird das rohrförmige Gewinnungsgerät mit einem Schlaghammer bis zu einer Tiefe von ungefähr 15 mm in den subchondralen Knochen getrieben. Danach wird der Treiber vorzugsweise um ungefähr 90° gedreht, um die Spongiosabasis zu frakturieren und um den Knochen-Knorpel-Stanzzylinder entnehmen zu können. Das rohrförmige Gewinnungsgerät wird anschließend von der Spenderstelle zurückgezogen, wobei der gewonnene Stanzzylinder im Inneren des Rohrs eingeschlossen ist.

Aufgabe der vorliegenden Erfindung ist es, ein Instrument zur autologen Chondrozytentransplantation bereitzustellen, mit dem ein Implantatvlies insbesondere auf arthroskopischem Wege implantiert werden kann.

Erfindungsgemäß wird die Aufgabe durch ein Instrument zur autologen Chondrozytentransplantation dadurch gelöst, dass die zumindest eine Öffnung auch mit Überdruck beaufschlagbar ist.

Aufgrund des Unterdrucks haftet das Implantatvlies an der distalen Endfläche des Stempels. Das Implantatvlies kann dann mittels des Stempels passend in den präparierten Knorpeldefekt eingefügt werden. Nach Einsetzen des Implantatvlieses wird die Beaufschlagung mit Unterdruck unterbrochen, der Stempel wird vom Implantatvlies abgehoben und dieses verbleibt in dem präparierten Knorpeldefekt.

Somit ist es mit einem solchen stempelartigen Instrument möglich, minival-invasiv passgenau durch ein Arthroskop hindurch ein Implantatvlies zur autologen Chondrozytentransplantation in einen Knorpeldefekt einzusetzen.

Nach dem Einsetzen des Implantatvlieses in den Knorpeldefekt und nach Unterbrechen des Unterdrucks könnte es vorkommen, dass das Implantatvlies aufgrund von Adhäsion an der distalen Endfläche des Stempels haften bleibt. Dieses kann z.B. erfolgen, wenn das Implantatvlies, bei dem es sich um biologisches Material handelt, feuchtgehalten werden muss.

Wird über die Öffnungen ein Überdruck auf das Implantatvlies in Richtung des Knorpeldefekts ausgeübt, wird die Trennung des

Implantatvlieses von dem Stempel unterstützt. Dadurch kann eine Trennung sichergestellt werden, ohne dass dabei die Lage des bereits gesetzten Implantatvlieses verändert wird.

Das Beaufschlagen mit Überdruck erfolgt dabei durch Bewerkstelligen eines Fluidflusses durch die Öffnungen, bei dem es sich z.B. um steriles Wasser, sterile Salzlösung oder sterile komprimierte Luft handeln kann.

In einer Ausgestaltung der Erfindung sind an der distalen Endfläche des Stempels mehrere Öffnungen vorgesehen.

Durch diese Maßnahme wird das Implantatvlies an mehr als einer Stelle der distalen Endfläche gehalten. Dadurch wird das Implantatvlies deutlich fester und gleichmäßiger haftend an der distalen Endfläche gehalten. Damit kann ein Abschälen des Implantatvlieses oder eine Faltenbildung vermieden werden.

In einer Ausgestaltung der zuvor genannten Maßnahme ist eine Vielzahl der Öffnungen im Bereich des Umfangs der distalen Endfläche des Stempels angeordnet.

Durch diese Maßnahme wird sichergestellt, dass die Ränder des Implantatvlieses an der distalen Endfläche des Stempels festgehalten werden und das Implantatvlies kann besonders sicher und passgenau in den präparierten Knorpeldefekt eingesetzt werden.

In einer weiteren Ausgestaltung der Erfindung weist das Instrument ferner eine Knorpelstanze zum Ausstanzen von Knorpelgewebe auf.

Eine Knorpelstanze ist ein besonders einfaches Instrument, um einen Knorpelschaden zu präparieren.

In einer weiteren Ausgestaltung der oben genannten Maßnahme ist die Knorpelstanze rohrförmig ausgebildet.

Durch diese Maßnahme kann eine Knorpelstanze nicht nur arthroskopisch verwendet werden, um den Knorpeldefekt auszustanzen, sondern bildet gleichzeitig einen Kanal, durch den weitere Instrumente an den Knorpeldefekt herangeführt werden können.

Zu diesen anderen Instrumenten können z.B. chirurgische Löffel oder Küretten zählen, die dazu dienen, das ausgestanzte Gewebe vom Knochen zu entfernen.

Ferner kann eine rohrförmig ausgebildete Knorpelstanze auch dazu dienen, durch diese den Stempel an den Knorpeldefekt heranzuführen. Sie kann also zusätzlich als Zielgerät für den Stempel wirken.

In einer weiteren Ausgestaltung der oben genannten Maßnahme entspricht der Innenquerschnitt der Knorpelstanze in etwa der Querschnittskontur des Stempels.

Mit dem Stempel kann so ein Implantatvlies eingeführt werden, das exakt der Stanzfläche entspricht.

Als geeignete Querschnittskontur hat sich ein ovalärer Querschnitt herausgestellt.

In einer weiteren Ausgestaltung der Erfindung weist das Instrument ferner zumindest eine Vliesstanze zum Ausstanzen des Implantatvlieses auf.

Mittels einer Vliesstanze kann ein Implantatvlies auf besonders einfache Weise präpariert werden. Die Stanzklinge der Vliesstanze weist dabei in etwa die gleiche Kontur wie die der Knorpelstanze auf, damit ein für den zuvor präparierten Defekt passendes Vliesstück ausgestanzt wird.

In einer Ausgestaltung der zuvor genannten Maßnahme ist die Vliesstanze rohrförmig ausgebildet.

Durch diese Maßnahme kann nach dem Stanzvorgang der Stempel durch die Vliesstanze an das ausgestanzte Implantatvlies herangeführt werden. Dies erleichtert das Übernehmen des Implantatvlieses durch den Stempel deutlich.

In einer weiteren Ausgestaltung der oben genannten Maßnahme entspricht der Innenquerschnitt der Vliesstanze in etwa der Querschnittskontur des Stempels.

Durch diese Maßnahme kann der Stempel im Passsitz in die Vliesstanze eingeführt und an das ausgestanzte Vliesstück herangeführt werden. Der Stempel wird zielgenau über dem ausgestanzten Implantatvlies positioniert und kann dieses übernehmen.

In einer weiteren Ausgestaltung der Erfindung sind unterschiedlich ausgeformte, untereinander angepasste Sätze an Stempeln, Vlies- und Knorpelstanzen vorhanden.

Durch diese Maßnahme kann ein Operateur aus mehreren Instrumentensätzen den Satz aussuchen der in Form und Größe dem zu behandelnden Defekt entspricht. Dabei zeigt sich, dass mit drei verschiedenen Größen etwa 90 % der gängigen Defektgrößen abgedeckt werden können.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Ansicht eines Stempels eines Instruments zur ACT,
- Fig. 2: eine perspektivische Ansicht einer Knorpelstanze eines Instruments zur ACT,
- Fig. 3: eine perspektivische Ansicht einer Vliesstanze eines Instruments zur ACT,
- Fig. 4: stark schematisch einen ersten Schritt eines Verfahrens zur ACT unter Verwendung eines erfindungsgemäßen Instruments,
- Fig. 5: stark schematisch einen zweiten Schritt des Verfahrens von Fig. 4,
- Fig. 6: stark schematisch einen dritten Schritt des Verfahrens von Fig. 4,
- Fig. 7: stark schematisch einen vierten Schritt des Verfahrens von Fig. 4,
- Fig. 8: stark schematisch einen fünften Schritt des Verfahrens von Fig. 4,
- Fig. 9: stark schematisch einen sechsten Schritt des Verfahrens von Fig. 4,
- Fig. 10: stark schematisch einen siebten Schritt des Verfahrens von Fig. 4,
- Fig. 11: stark schematisch einen achten Schritt des Verfahrens von Fig. 4,
- Fig. 12: stark schematisch einen neunten Schritt des Verfahrens von Fig. 4, und
- Fig. 13: stark schematisch einen zehnten Schritt des Verfahrens von Fig. 4.

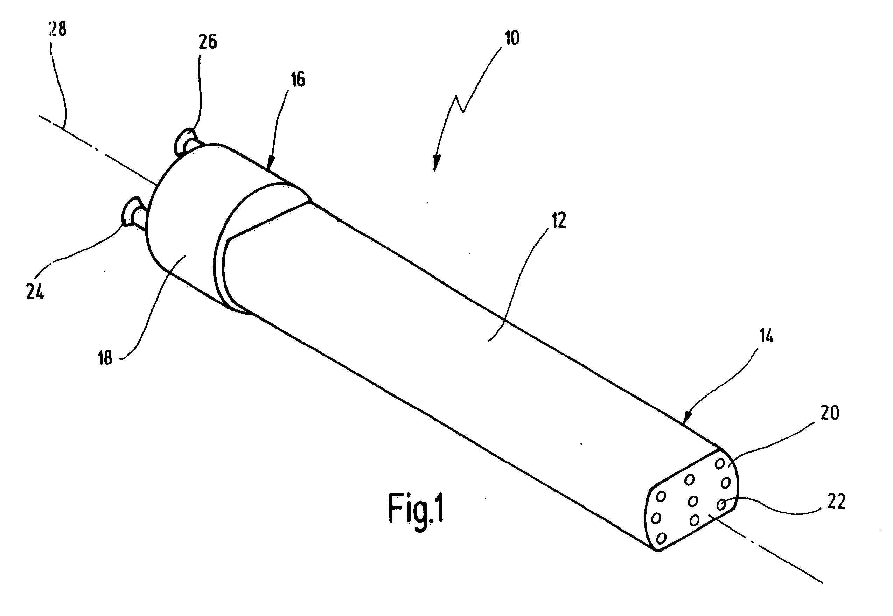

In Fig. 1 ist ein Stempel eines Instruments zur ACT in seiner Gesamtheit mit der Bezugsziffer 10 bezeichnet.

Der Stempel 10 weist einen stabförmigen Körper 12 mit einem distalen Abschnitt 14 und einem proximalen Abschnitt 16 auf.

Der proximale Abschnitt 16 weitet sich dabei zu einem Griff 18 auf.

Der distale Abschnitt 14 endet in einer planen Endfläche 20, die grob die Form eines Rechtecks mit abgerundeten Ecken aufweist. Diese etwa ovaläre Form ist für die ACT besonders geeignet, da die meisten Knorpeldefekte in Form von Rissen vorliegen, so dass in den meisten Fällen ein etwa ovaläres Implantat eingesetzt werden muss.

In der Endfläche 20 sind Öffnungen 22 ausgespart, die gleichmäßig verteilt und insbesondere im Bereich des Umfangs angeordnet sind. Mittels dieser Öffnungen 22 kann ein Implantatvlies an den Stempel 10 angesaugt werden. Die Anordnung der Öffnungen 22 sorgt dabei dafür, dass die Ränder des Implantatvlieses fest haftend an den Stempel 10 angesaugt werden. Die Öffnungen 22 sind über Bohrungen im Körper 12 mit einem ersten Anschluss 24 und einem zweiten Anschluss 26 am proximalen Abschnitt 16 des Körpers 12 des Stempels 10 verbunden.

Der erste Anschluss 24 kann dabei mit einer Quelle für einen Unterdruck verbunden werden. Dadurch können die Öffnungen 22 mit einem Unterdruck beaufschlagt und ein Implantatvlies an die Endfläche 20 des Stempels 10 angesaugt werden.

Der zweite Anschluss 26 kann mit einer Quelle für ein steriles Fluid verbunden werden, wodurch eine Strömung in Richtung des distalen Abschnitts 14 des Körpers 12 erzeugt werden kann. Durch diese Strömung kann ein Implantatvlies von der Endfläche 20 des Stempels 10 gelöst werden. Ein hier nicht dargestelltes Ventil erlaubt das Umschalten in die unterschiedlichen Betriebsmodi.

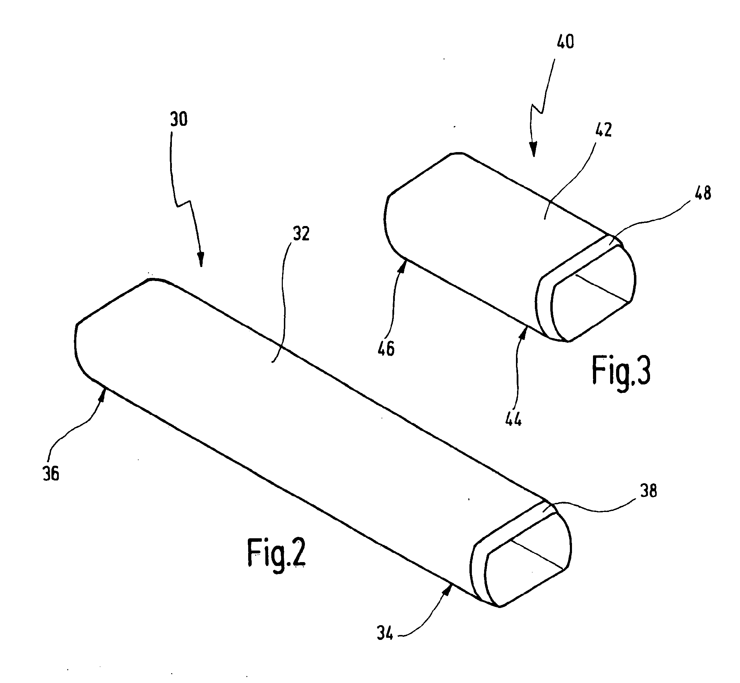

In Fig. 2 ist eine Knorpelstanze in ihrer Gesamtheit mit der Bezugsziffer 30 bezeichnet.

Die Knorpelstanze 30 weist einen Körper 32 auf, der rohrförmig ausgebildet ist. Der Innenquerschnitt des Körpers 32 entspricht dabei der Querschnittskontur des Körpers 12 des Stempels 10 von Fig. 1.

Der Körper 32 weist einen distalen Abschnitt 34 und einen proximalen Abschnitt 36 auf. Der distale Abschnitt 34 ist zu einer Stanzklinge 38 angeschliffen, die sich über den gesamten Umfang des Körpers 32 erstreckt.

In Fig. 3 ist eine Vliesstanze eines Instruments zur ACT in ihrer Gesamtheit mit der Bezugsziffer 40 bezeichnet.

Die Vliesstanze 40 weist einen rohrförmigen Körper 42 mit einem distalen Abschnitt 44 und einem proximalen Abschnitt 46 auf.

Der Körper 42 entspricht in Aufbau und Durchmesser im Wesentlichen dem Körper 32 der Knorpelstanze 30 von Fig. 2, wobei der distale Abschnitt 44 ebenfalls zu einer vollumfänglichen Stanzklinge 48 angeschliffen ist.

Die Stanzklinge 48 weist dabei die gleiche Form und Größe auf wie die Stanzklinge 38 der Knorpelstanze 30 von Fig. 2.

Durch diese Dimensionierung wird sichergestellt, dass mit der Vliesstanze 40 ein Implantatvlies ausgestanzt wird, das die gleiche Form und Größe aufweist wie der von der Knorpelstanze 30 ausgestanzte Knorpelbereich.

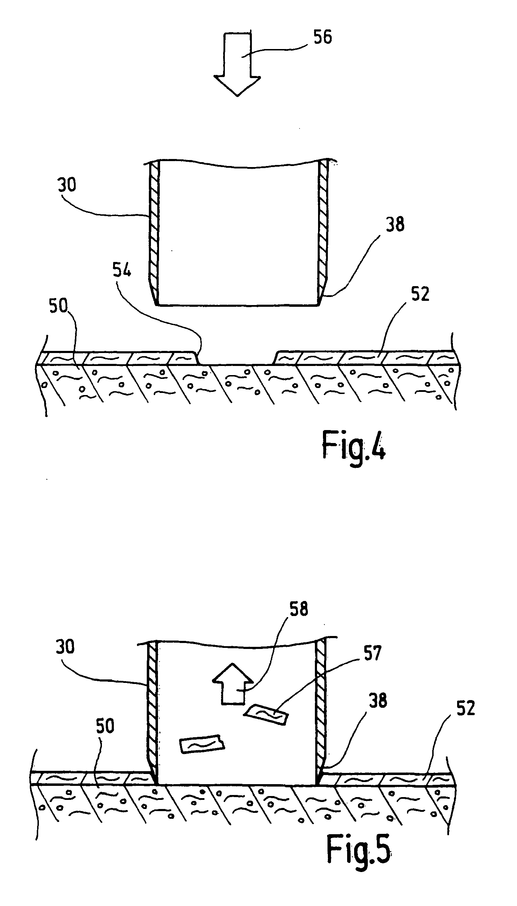

In Fig. 4 ist schematisch ein erster Schritt eines Verfahrens zur ACT dargestellt.

Hierbei wird die Knorpelstanze 30 an einen Knochen 50 herangeführt, der von einem Knorpel 52 bedeckt ist.

Der Knochen 50 befindet sich im Kniegelenk. Die Oberfläche des Knochens 50 ist nur aus Gründen der Einfachheit eben dargestellt. Im Kniegelenk werden fast ausschließlich gekrümmte Knochenoberflächen angetroffen.

Die zur Einführung der Knorpelstanze 30 verwendeten arthroskopischen Instrumente, wie z.B. ein Arthroskop zur Sichtkontrolle, sind aus Gründen der Einfachheit ebenfalls nicht dargestellt.

Der Knorpel 52 weist einen Knorpeldefekt 54 auf, der hier in Form eines Verlusts von Knorpelgewebe auftritt.

Die Stanzklinge 38 ist um den Knorpeldefekt 54 ausgerichtet.

Die Knorpelstanze 30 wird nun in Richtung eines Pfeils 56 an den Knochen 50 und den Knorpel 52 herangeführt. Die Stanzklinge 38 wird dabei in den Knorpel 52 eingedrückt und durchtrennt diesen, wodurch die Ränder des Knorpeldefekts 54 ausgestanzt werden.

In Fig. 5 ist dargestellt, dass die Stanzklinge 38 der Knorpelstanze 30 den Knorpel 52 durchtrennt hat und gegen die Oberfläche des Knochens 50 zu liegen kommt.

Die durch die Stanzklinge 38 ausgestanzten Stücke 57 des Knorpels 52 werden mittels eines hier nicht dargestellten chirurgischen Löffels in Richtung eines Pfeils 58 aus der Knorpelstanze 30 entfernt.

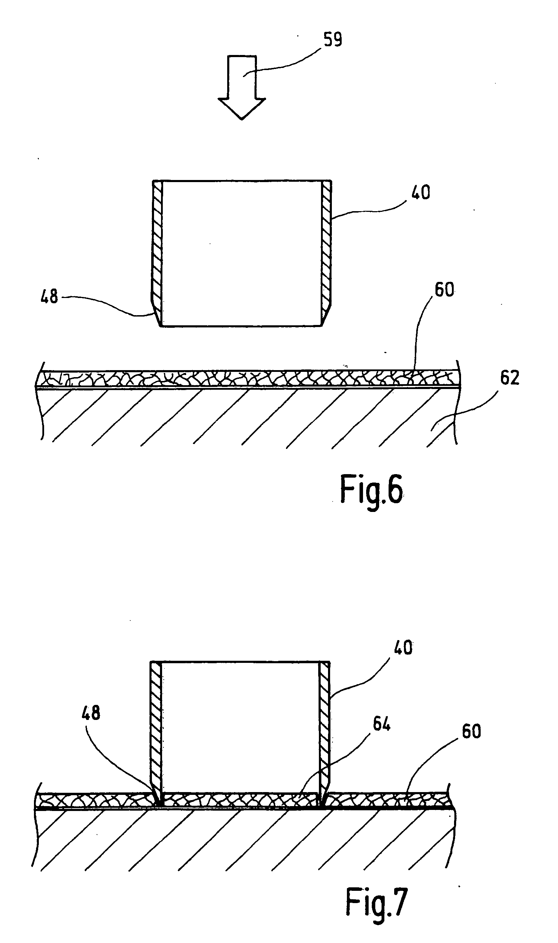

In Fig. 6 ist ein weiterer Schritt des Verfahrens zur ACT dargestellt. Dieser und die folgenden Verfahrensschritte zur Präparation eines Implantatvlieses können vor, während oder nach den zuvor gezeigten zwei Verfahrensschritten durchgeführt werden.

Hierbei wird die Vliesstanze 40 in Richtung eines Pfeils 59 auf ein Vlies 60 zubewegt und in dieses hineingedrückt.

Bei dem Vlies 60 handelt es sich um eine Schweinecollagenmatrix, auf der zuvor im Labor Chondrozyten eines zu behandelnden Patienten angezüchtet wurden. Die Dicke des Vlieses 60 entspricht in etwa der Dicke des zu behandelnden Knorpels 52. Das Vlies 60 liegt auf einer Unterlage 62, hier einer Metallplatte, die fest genug ist, um dem Stanzvorgang einen Widerstand entgegenzusetzen.

Wie aus Fig. 7 ersichtlich wird, wurde die Stanzklinge 48 in das Vlies 60 hineingedrückt und hat ein Implantatvlies 64 aus diesem herausgetrennt. Durch die Dimensionierung der Vliesstanze 40 und der Knorpelstanze 30 hat das Implantatvlies 64 die gleiche Größe wie der zuvor durch die Knorpelstanze 30 ausgestanzte Bereich des Knorpels 52.

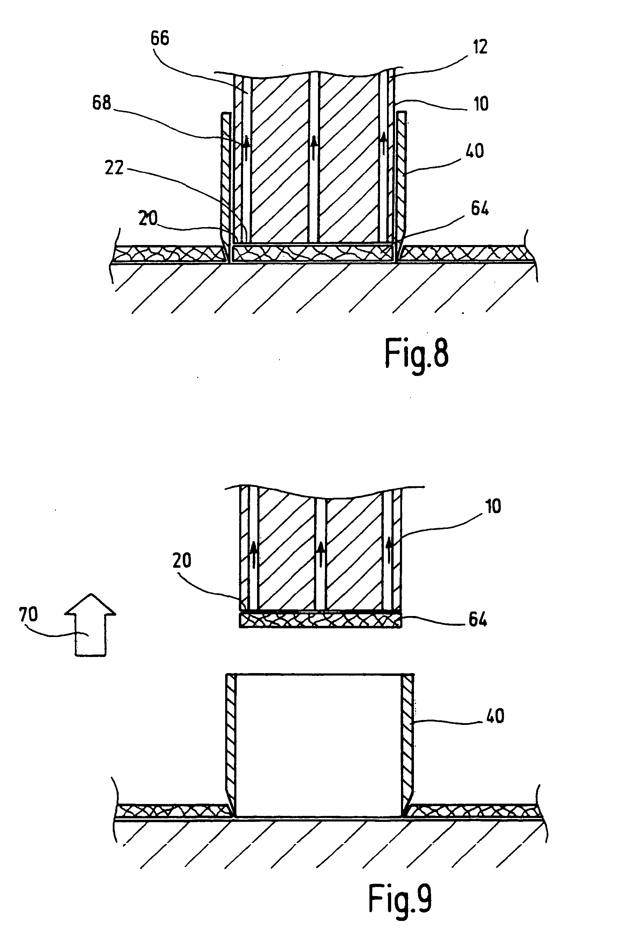

In die Vliesstanze 40 wird jetzt, wie in Fig. 8 dargestellt, aus proximaler Richtung der Stempel 10 so weit eingeführt, dass die Endfläche 20 des Stempels 10 auf dem Implantatvlies 64 zum Liegen kommt.

Durch die Dimensionierung des Stempels 10 und der Vliesstanze 40 wird der Stempel 10 im Passsitz in die Vliesstanze 40 eingeführt. Somit wird sichergestellt, dass die Endfläche 20 genau über dem Implantatvlies 64 zu liegen kommt.

Jetzt wird in Bohrungen 66 im Körper 12 des Stempels 10 ein Unterdruck in Richtung der Pfeile 68 angelegt.

Die Bohrungen 66 sind mit den Öffnungen 22 in der Endfläche 20 des Stempels 10 verbunden. Durch diesen Unterdruck wird das zuvor ausgestanzte Implantatvlies 64 an die Endfläche 20 des Stempels 10 angesaugt.

Wenn das Implantatvlies 64 an die Endfläche 20 des Stempels 10 angesaugt ist, kann dieser, wie in Fig. 9 dargestellt, in Richtung eines Pfeils 70, also in proximaler Richtung aus der Vliesstanze 40 abgezogen werden. Hierbei wird das Implantatvlies 64 aus der Vliesstanze 40 entnommen und bleibt sicher an der Endfläche 20 des Stempels 10 haften. Dies erlaubt die weitere Handhabung des Zusammenbaus aus Stempel 10 und Implantatvlies 64, wobei letzteres fest haftet.

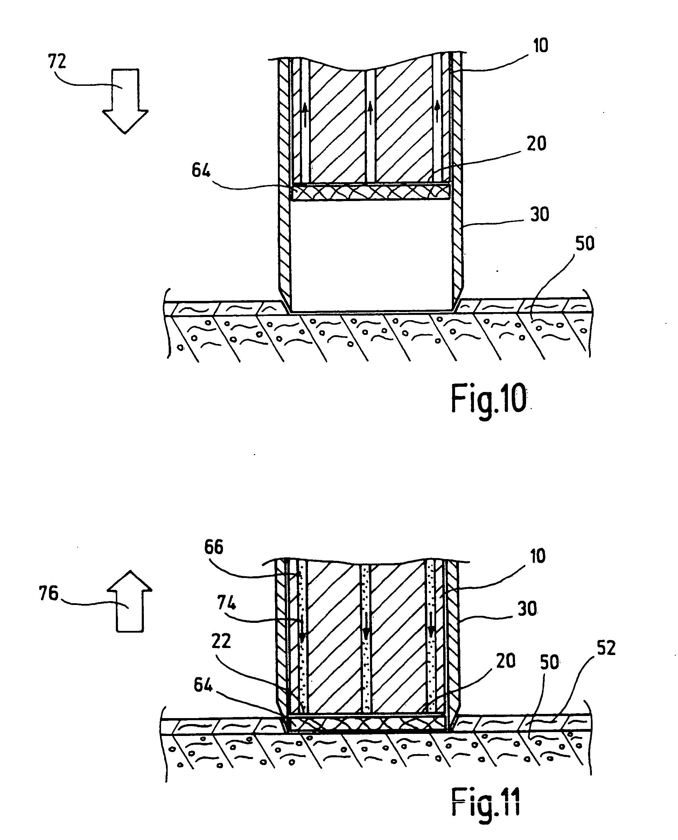

Der Stempel 10 samt anhaftendem Implantatvlies 64 wird nunmehr von proximal in die noch im Knorpel 50 steckende Knorpelstanze 30 eingeführt, wie das aus Fig. 10 ersichtlich ist.

Das Implantatvlies 64 wird, wie in Fig. 11 dargestellt, in den präparierten Knorpeldefekt eingebracht und liegt an der Oberfläche des Knochens 50 an. Es schließt dabei von seiner Höhe etwa mit dem Knorpel 52 ab. In einem nächsten Schritt wird nun in den Bohrungen 66 ein Fluidfluss in Richtung der Pfeile 74 erzeugt. Dies geschieht hier durch Einleiten von sterilem Wasser. Durch diesen Fluidfluss wird an den Öffnungen 22 ein Überdruck erzeugt, der ein Ablösen des Implantatvlieses 64 von der Endfläche 20 des Stempels 10 fördert.

Der Stempel 10 wird anschließend in Richtung eines Pfeils 76 aus der Knorpelstanze 30 abgezogen werden.

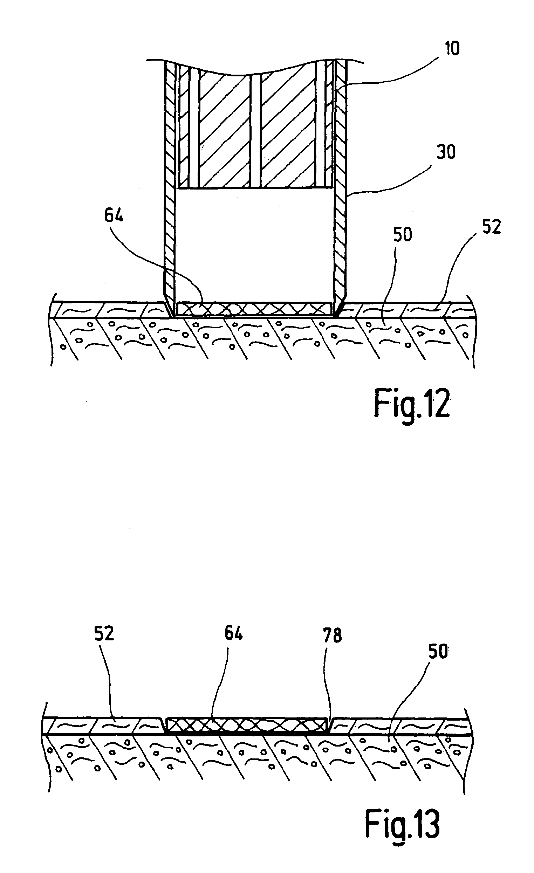

Das Implantatvlies 64 verbleibt, wie in Fig. 12 dargestellt, an der Oberfläche des Knochens 50 anliegend während der Stempel 10 bereits weitgehend aus der Knorpelstanze 30 abgezogen wurde. In einem auf das Abziehen des Stempels folgenden Schritt wird nun auch die Knorpelstanze 30 aus dem Körper eines Patienten entnommen.

In Fig. 13 ist die Situation nach Abschluss des Verfahrens zur ACT dargestellt.

Nach der Entnahme des Stempels 10 und der Knorpelstanze 30 verbleibt alleine das Implantatvlies 64 im Körper des Patienten. Dieses liegt nun bündig an der Oberfläche des Knochens 50 an und weist etwa die gleiche Höhe wie der Knorpel 52 auf. An den Stellen, an denen die Klinge 38 der Knorpelstanze 30 in den Knorpel 52 eingedrungen ist, verbleibt noch ein kleiner Spalt 78, an dem das Implantatvlies 64 mittels Fibrinkleber mit dem Knorpel 52 verklebt werden kann.

Im Laufe des Heilungsprozesses wachsen die im Implantatvlies 64 vorhandenen Knorpelzellen und verbinden sich mit dem bereits vorhandenen Knorpel 52. Die Matrix des Implantatvlieses 64, also das Schweinecollagen wird gleichzeitig abgebaut. Nach einer gewissen Zeit ist das Implantatvlies 64 dann vollständig durch neues Knorpelgewebe ersetzt und der Knorpelschaden vollkommen beseitigt.

Zur Förderung des Anwachsens des Implantatvlieses 64 kann mittels eines Instrumentes, wie es in der Anmeldung EP-A-1 698 285 mit dem Titel "Medizinisches Instrument zum Einbringen von Mikrofrakturen in Knochen" beschrieben ist, ein "Microfracturing" am in Fig. 5 freigelegten Knochen durchgeführt werden. Das aus den Mikrofrakturen austretende Gerinnsel kann sich im Implantatvlies verteilen und Knorpelgewebe ausbilden.

## Patentansprüche

1. Medizinisches Instrument zur autologen Chondrozytentransplantation, mit einem Stempel (10) zum Einbringen eines Implantatvlieses (64) in einen Knorpel (52), der an einer distalen Endfläche (20) zumindest eine Öffnung (22) aufweist, die mit einem Unterdruck beaufschlagbar ist, so dass ein Implantatvlies (64) an der Endfläche (20) haltbar ist, **dadurch gekennzeichnet, dass** die zumindest eine Öffnung (22) mit Überdruck beaufschlagbar ist.

2. Instrument nach Anspruch 1, **dadurch gekennzeichnet, dass** an der distalen Endfläche (20) des Stempels (10) mehrere Öffnungen (22) vorgesehen sind.

3. Instrument nach Anspruch 2, **dadurch gekennzeichnet, dass** eine Vielzahl der Öffnungen (22) im Bereich des Umfangs der distalen Endfläche (20) des Stempels (10) angeordnet ist.

4. Instrument nach einem der Ansprüche 1 bis 3, **dadurch gekennzeichnet, dass** es ferner zumindest eine Knorpelstanze (30) zum Ausstanzen von Knorpelgewebe aufweist.

5. Instrument nach Anspruch 4, **dadurch gekennzeichnet, dass** die zumindest eine Knorpelstanze (30) rohrförmig ausgebildet ist.

6. Instrument nach Anspruch 5, **dadurch gekennzeichnet, dass** der Innenquerschnitt der Knorpelstanze (30) in etwa der Querschnittskontur des Stempels (10) entspricht.

7. Instrument nach einem der Ansprüche 4 bis 6, **dadurch gekennzeichnet, dass** es ferner zumindest eine Vliesstanze (40) zum Ausstanzen des Implantatvlieses (64) aufweist.

8. Instrument nach Anspruch 7, **dadurch gekennzeichnet, dass** die zumindest eine Vliesstanze (40) rohrförmig ausgebildet ist.

9. Instrument nach Anspruch 8, **dadurch gekennzeichnet, dass** der Innenquerschnitt der Vliesstanze (40) in etwa der Querschnittskontur des Stempels (10) entspricht.

10. Instrument nach einem der Ansprüche 7 bis 9, **dadurch gekennzeichnet, dass** unterschiedlich ausgeformte, untereinander angepasste Sätze an Stempeln (10), Knorpelstanzen (30) und Vliesstanzen (40) vorhanden sind.

## Claims

1. Medical instrument for autologous chondrocyte transplantation, comprising a stamp (10) for introducing an implantable nonwoven (64) into a cartilage (52), having a distal end face (20), at least one opening (22) being provided in said distal end face (20), to which opening (22) an underpressure can be applied for holding an implantable nonwoven (64) at the end face (20), **characterized in that** the at least one opening (22) can be subjected to an overpressure.

2. Instrument of claim 1, **characterized in that** a plurality of openings (22) are provided in said distal end face (20) of the stamp (10).

3. Instrument of claim 2, **characterized in that** a multiplicity of said openings (22) are arranged in an area of a circumference of said distal end face (20) of said stamp (10).

4. Instrument of anyone of claims 1 through 3, **characterized in that** it further comprises at least one cartilage puncher (30) for punching out a cartilage tissue.

5. Instrument of claim 4, **characterized in that** the at least one cartilage puncher (30) has a tubular configuration.

6. Instrument of claim 5, **characterized in that** an inner cross section of the cartilage puncher (30) corresponds approximately to a cross-sectional contour of the stamp (10).

7. Instrument of anyone of claims 4 through 6, **characterized in that** it further comprises at least one nonwoven puncher (40) for punching out the implantable nonwoven (64).

8. Instrument of claim 7, **characterized in that** the at least one nonwoven puncher (40) has a tubular configuration.

9. Instrument of claim 8, **characterized in that** the inner cross section of the nonwoven puncher 40 corresponds approximately to the cross-sectional contour of the stamp (10).

10. Instrument of anyone of claims 7 through 9, **characterized in that** different shaped sets of stamps (10), cartilage puncher (30) and nonwoven punchers (40) are provided, which are adapted to one another.

## Revendications

1. Instrument médical pour la transplantation de chondrocytes autologues, comprenant un tampon (10) permettant d'introduire un non-tissé implantable (64) dans un cartilage (52), qui présente sur une surface terminale distale (20) au moins une ouverture (22) qui peut être soumise à une dépression, de façon qu'un non-tissé implantable (64) puisse être maintenu sur la surface terminale (20), **caractérisé en ce que** la dite au moins une ouverture (22) peut être soumise à une surpression.

2. Instrument selon la revendication 1, **caractérisé en ce que** plusieurs ouvertures (22) sont prévues sur la surface terminale distale (20) du tampon (10).

3. Instrument selon la revendication 2, **caractérisé en ce qu'**une pluralité d'ouvertures (22) sont disposées dans la région de la périphérie de la surface terminale distale (20) du tampon (10).

4. Instrument selon une des revendications 1 à 3, **caractérisé en ce qu'**il présente en outre au moins un poinçon à cartilage (30) pour poinçonner du tissu cartilagineux.

5. Instrument selon la revendication 4, **caractérisé en ce que** ledit au moins un poinçon à cartilage (30) est conformé en tube.

6. Instrument selon la revendication 5, **caractérisé en ce que** la section intérieure du poinçon à cartilage (30) correspond approximativement au contour de section du tampon (10).

7. Instrument selon une des revendications 4 à 6, **caractérisé en ce qu'**il présente en outre au moins un poinçon à non-tissé (40) pour poinçonner le non-tissé implantable (64).

8. Instrument selon la revendication 7, **caractérisé en ce que** ledit au moins un poinçon à non-tissé (40) est conformé en tube.

9. Instrument selon la revendication 8, **caractérisé en ce que** la section intérieure du poinçon à non-tissé (40) correspond approximativement au contour de section du tampon (10).

10. Instrument selon une des revendications 7 à 9, **caractérisé en ce qu'**il est prévu des jeux de tampons (10), de poinçons à cartilage (30) et de poinçons à non-tissé (40) de forme différente, adaptés les uns aux autres.
